# EUROPEAN PATENT SPECIFICATION

(11) **EP 0 446 273 B1**
(45) Date of publication and mention of the grant of the patent: **14.10.1998**
(21) Application number: 90900599.3
(22) Date of filing: 05.12.1989
(51) Int. Cl.: C12P 21/08, C07K 14/155, G01N 33/577, G01N 33/569

(54) **MONOCLONAL ANTIBODIES TO FELINE-T-LYMPHOTROPIC LENTIVIRUS**
MONOKLONALE ANTIKÖRPER GEGEN KATZEN-T-LYMPHOTROPES LENTIVIRUS
ANTICORPS MONOCLONAUX CONTRE LE LENTIVIRUS LYMPHOTROPIQUE T

(30) Priority: 05.12.1988 US 279989; 05.01.1989 US 293906
(43) Date of publication of application: 18.09.1991
(73) Proprietor: IDEXX LABORATORIES, INC., Portland, ME 04101 (US)
(72) Inventor: O'CONNOR, Thomas, P., Westbrook, ME 04092 (US); TONELLI, Quentin, J., Portland, ME 04103 (US)
(74) Representative: Deans, Michael John Percy
(86) International application number: PCT/US89/05492
(87) International publication number: WO 90/06510

(56) References cited:
- US-A- 4 514 505
- JOURNAL OF VETERINARY MEDICINE vol. 35, no. 10, 1 December 1988, BERLIN FRG pages 773 - 778; H. LUTZ ET AL.: 'Specific assessment of feline T-lymphotropic lentivirus serology.'
- AMERICAN JOURNAL OF VETERINARY RESEARCH vol. 49, no. 8, 1 August 1988, WASHINGTON DC USA pages 1246 - 1258; J.K. YAMAMOTO ET AL.: 'Pathogenesis of experimentally induced feline immunodeficiency virus infection in cats.'
- JOURNAL OF CLINICAL MICROBIOLOGY vol. 28, no. 5, 1990, WASHINGTON DC USA pages 898 - 904; G.K. TILTON ET AL.: 'Immunoassay for detection of feline immunodeficiency virus core antigen.'
- K. HELLSTROM, "Monoclonal Anti-melanoma Antibodies and Their Possible Clinical Use" in "Monoclonal Antibodies For Cancer Detection and Therapy", published 1985, by Academic Press Inc. (London), see page 20.
- Science, Volume 235, issued 1987 February (Washington, D.C.), N.C. PEDERSON, "Isolation of a T-Lymphotropic Virus from Domestic Cats with an Immumodeficiency-Like Syndrome", see page 793-Figure 5.
- Diagnostic Horizons, Volume 2, No. 1, issued 1978 February (Walkersville, Maryland), A. VOLLER, "The Enzyme Linked Immunosorbent Assay (ELISA)", see page 3.

## Description

This invention relates to monoclonal antibodies to feline-T-lymphotropic lentivirus (also called feline immunodeficiency virus, FIV), to related polypeptides and to use of these monoclonals for detection and purification of the virus.

Pederson et al., 235 Science 790, 1987, describes detection and isolation of FIV from domestic cats having an immunodeficiency-like syndrome. The virus was purified by centrifugation on sucrose gradients in Tris base pH 7.4 containing 0.1 M NaCl and 1 mM EDTA. Western blots prepared from gradient purified virus and their reaction with sera from experimentally infected cats was determined. A few protein bands were detected and "[a]lthough antigenic comparison was not made, the position of these bands may correspond to the major core protein p24, gag precursor protein p55 and endonuclease protein p32 of HIV".

J. Veterinary Medicine, 35 (December 1, 1988) pages 773 - 778 and American Journal of Veterinary Research, 49 (August 1, 1988) page 1246 - 1258 both describe Feline-T-Lymphotropic Lentivirus, identifying a number of FTLV proteins, and antibodies which recognise these proteins.

According to the invention, we provide a purified polypeptide comprising an epitope of a feline immunodeficiency virus (FIV) protein, said FIV protein being characterised in that it is:
a) an FIV envelope protein obtainable from FIV-infected cells;
b) approximately 130 kD in weight;
c) binds to monoclonal antibody produced by hybridoma cell line 2F11 (American Type Culture Collection (ATCC) Deposit Number HB 10295); and
d) binds to serum of FIV-infected cats.

In a second aspect the invention features monoclonal antibodies specific for an epitope of an FIV-encoded antigen, e.g., a protein, polypeptide, or glycoprotein. By monoclonal antibody is meant any antibody that is produced from a single antibody-producing cell which has been cloned to produce an antibody-producing cell line. It does nct include the various antibodies found in a polyclonal preparation, i.e., a preparation produced by inoculation of an animal,with an antigen and recovery of the resulting serum. By epitope is meant any specific amino acid sequence, modified amino acid sequence, or protein secondary or tertiary structure which is recognized by an antibody. This epitope may be present on one or more antigens of FIV, but is not present in any of the cther components commonly associated with FIV, for example, feline cells and serum.

We also describe a solution containing a plurality of monoclonal antibodies in addition to the monoclonal antibodies of the invention, each antibody being specific for an epitope of an FIV-encoded antigen.

We also describe below a method for detecting an FIV envelope glycoprotein antigen in a sample, said method comprising the steps of:
a) providing an antibody that specifically recognises a polypeptide as defined above;
b) contacting said antibody with said sample under conditions in which said antibody forms a complex with a sample antigen; and
c) detecting said complex, wherein the presence of said complex indicates the presence of said glycoprotein in said sample.

In preferred embodiments, the epitope is present on an antigen selected frcm the p10, p15, p26, p47, p110, gp40 or gp130 antigens of FIV (i.e., those FIV-encoded antigens with molecular weights of 10, 15, 26, 40, 50, 110 or 130 kD as described below); the antibody has sufficient affinity for the epitope to selectively identify the epitope in an immunoassay for FIV; the antibody recognizes an epitope on the FIV viral particle; the antibody is conjugated with a detectable label, the label not significantly interfering with the specificity or affinity of the antibody for the epitope, most preferably the label is an enzyme, e.g., horseradish peroxidase; and the antibody is produced by a cell line deposited with the American Type Culture Collection, and assigned the number HB9888, HB9889, or HB9890.

Our monoclonal antibodies are suitable for use in assays and purification of FIV. Applicant has determined a method by which sufficiently pure FIV antigen can be prepared in order to allow these monoclonal antibodies to be isolated, and to detect the presence of such monoclonal antibodies in a screening procedure. Applicant has also described means by which useful antibodies can be distinguished from those which are not useful.

Other features and advantages of the invention will be apparent from the following description of the preferred embodiments thereof.

In the drawings:
Fig. 1 is a photograph of the major viral associated proteins of FIV identified by polyacrylamide gel electrophoresis (PAGE) and stained with Commassie Blue R250 (lane A); molecular weight standards are shown in lane B; and
Fig. 2 is a photograph of a Western immunoblot analysis of antibodies to FIV found in serum from cats identified as positive by an ELISA assay for FIV antibodies.

### FIV Antigen

The FIV antigen is derived from FIV-infected cells, and takes the form of FIV virus particles, or is derived from FIV virus particles, as follows. (Synthetic antigens are also suitable in the assay, as are substantially purified polypeptides derived from FIV particles.) Master seed virus producing cultures were obtained in the form of a continuous feline cell line infected with FIV isolate #2427 (CRFK-FIV or Petaluma strain) from Dr. Neils Pederson (University of California, Davis, California). The parent cell line is Crandell feline kidney cell persistently infected with FIV. The cell line was deposited with the American Type Culture Collection on July 13, 1988 and assigned the number CRL9761. The deposit was made under the Budapest Treaty.

Other virus cultures can be obtained as described by Pederson, supra, or by Harbour et al., 122 The Veterinary Record 84, 1988. Seed stocks of virus producing cell cultures were obtained by freeze-downs of FIV-infected master seed cell cultures following at least 19 post infection passages in culture. Additional seed stocks of virus producing cultures were obtained by either infection of the continuous feline cell culture with FIV master seed virus or by single cell microwell cloning of high level FIV producers from the original FIV infected master seed cell culture. For propagation, master seed virus infected feline cell cultures were inoculated into tissue cell culture flasks. Following growth to a confluent monolayer of cells, tissue culture fluid was harvested at intervals of 2-5 days.

Working seed virus was produced by propagation by the master seed cell line permanently infected with FIV. An inoculum was added to tissue culture flasks, in Dulbecco's Modified Eagles medium containing 2 mM L-glutamine and 4.5 g per liter/glucose (DME) containing 100 units per ml. penicillin and streptomycin and 2 mM glutamine. An inoculum was added to tissue culture flasks, incubated, and the spent tissue culture fluid harvested when the cells were grown to confluence. The cells were released from the culture vessel with trypsin/EDTA and diluted between 1:5 and 1:25 (typically 1:8) in medium. Typically the flasks were incubated at 36°C-38°C for a maximum of 7 days (between 3 and 7 days) before fluid and cell harvest. The harvested fluid, including cell material, was centrifuged in a high speed centrifuge (Sorval RC-5B or Beckman J2-21) leading to separation of supernatant and cell pellet material. The cell pellet was discarded, and the supernatant culture fluid used to prepare working virus. The clarified supernatant was made 0.5 M in NaCl and 4%-10% (usually 7%) in polyethylene glycol (PEG 8000, Sigma). Following overnight incubation at 2°C-7°C, virus was pelleted (at 13,000 x g for 30 min.) and resuspended in buffer (10 mM Tris, pH 7.6 300 mM NaCl, 1mM EDTA, at 2°C-7°C). After overnight incubation the virus was centrifuged at 13,000 x g for 15 min., the pellet discarded and the supernatant centrifuged on a 50%/80% discontinuous glycerol step gradient in 10 mM tris 300 mM NaCl, 1 mM EDTA at pH 7.6. Centrifugation was at 100,000 x g for 3 hrs, at 4°C and the FIV viral band at the 50%-80% interface collected. The band was suspended in 10 mM Tris, 0.3 M NaCl and 1 mM EDTA and diluted 1:3 in the buffer and repelleted at 100,000 x g for 1 hr. The resulting pellet was purified virus and was resuspended in the above buffer and stored at -70°C. The resulting virus was substantially free from FIV host cell proteins and was composed of at least 5% p26 (the major nucleocapsid protein, as measured by densitometric scans of Commassie Blue 250 stained SDS/PAGE as total protein), Such antigen is sufficiently pure for preparation of monoclonal antibodies and for screening for suitable monoclonal antibodies.

Such purified antigen may be obtained by other techniques, however, applicants have found that high molecular weight contaminants present in virus preparations may be eliminated by use of the high salt (i.e., greater than physiological range salt concentration) used in the gradient centrifugation procedure.

### FIV Glycoprotein

In order to prepare monoclonal antibodies to viral glycoproteins, antigenic glycoprotein was firstly prepared as follows.

Actively growing CRFK FTLV infected ceils were scraped from roller bottles, gently washed with phosphate-buffered saline (PBS), and pelleted. The cell pellet was gently resuspended in 10 mM sodium phosphate, pH 7.2, at a ratio of 1 ml buffer to 0.1 ml of cell pellet. This suspension was incubated on ice or refrigerated for 5-10 min., vigorously vortex mixed for 30 seconds, and four volumes of PBS with 1mM PMSF added. The mixture was then vigorously homogenized for 90-120 seconds with a Brinkmann Homogenizer PT10/35 with a PTA 20 generator.

The resulting homogenate was clarified for 20 minutes at 5,000g. The supernatant fraction was discarded and the cell membrane pellet resuspended in PBS + 0.2% Triton X-100 (Trade Mark) at a ratio of 2.5ml buffer to 0.1 ml original cell pellet. The mixture was then vigorously homogenized for 90-120 seconds with a Brinkmann Homogenizer PT 10/35 with a PTA 20 generator. The resulting homogenate was clarified at 100,000g for 1 hr, the supernatant decanted off and batch bound overnight at 20-23°C on Pharmacia Lentil Lectin Sepharose 4B at a ratio of 6 ml of resin to 5 ml of original cell pellet.

The Lentil Lectin Suspension was poured through a column, the resin collected, and washed with 15 column volumes of PBS + 0.2% Triton X-100. The glycoproteins were then eluted from the resin by subjecting the resin to 5-10 column volumes of PBS + 0.2% Triton X-100 + 200 mM methyl α-D mannopyranoside, collecting fractions of 1 column volume/tube.

The isolation of glycoproteins was verified by 9% SDS-PAGE electrophoresis, and checked using ³⁵S-radiolabeled cell preparations in conjunction with RIPA data.

Further purification of viral glycoprotein from host cell glycoprotein includes use of the HPLC or use of a polyclonal antibody for affinity chromotography.

### Preparation of FIV Monoclonal Antibodies

Balb/CJ (Jackson Labs) mice were immunized with an initial injection of 50 micrograms of FIV antigen per mouse mixed 1:1 with Difco Bacto adjuvant complete. After two weeks a booster injection of 100 micrograms of FIV antigen was injected into each mouse intravenously without adjuvant. Three days after the booster injection a fusion was performed with mouse myeloma cell lines FO or p3x63-Ag8 653. Mid log phase myeloma lines were harvested on the day of fusion and checked for viability. The cells were spun at 300 x g for 8 min., separated from the growth medium, and resuspended in serum free DME.

For fusion, an FIV-inoculated mouse was killed by cervical dislocation and the spleen aseptically removed. The spleen was washed three times in serum free DME and placed in a sterile Petri dish containing 20 mls of complete medium (DME containing 20% bovine fetal serum, 100 units per ml. of penicillin and streptomycin, and 1 mM sodium pyruvate). To release cells, the spleen was perfused with a 23 gauge needle.

Cells were placed in a 50 ml conical centrifuge tube and pelleted at 300 x g for 8 min. The pellet resuspended in 5 ml of 0.17M ammonium chloride and placed on ice for 8 min. 5 ml of bovine fetal serum (20%) was added and the cells pelleted again at 300 x g for 8 min. After resuspension in 10 ml DME the cells were counted and the spleen and myeloma cells mixed in a ratio of 3:1. The cell mixture was pelleted at 200 x g for 10 minutes, the supernatant decanted, and the pellet allowed to stand for 5 min. Over a period of 1 min., 1 ml of 50% PEG (PEG 1500 mixed 1:1 with Hepes pH 8.1) at 37°C was added. After 1 min. incubation at 37°C, 1 ml of DME was added over a period of another 1 min. and then a second 1 ml of serum free medium added over a period of 1 min. Finally, 10 mls of DME was added over a period of 2 min., the cells pelleted at 200 x g for 8 min., and the pellet resuspended in complete medium containing 0.016 mM thymidine, 0.1 mM hypoxanthine, 0.5 micromolar aminopterin, and 10% hybridoma cloning factor (1 x HAT). The cells were plated into 96-well plates.

After 3, 5 and 7 days half of the medium in the fusion plates was removed and replaced with fresh 1 x HAT. After 11 days the hybridoma cell supernatant was screened by an ELISA test. In this test, 96 well plates were coated with FIV antigen by standard technique. One hundred microliters of supernatant from each well was added to a corresponding well on a screening plate and incubated for 1 hr. at 20-22°C. After incubation, each well was washed three times with distilled water and 100 microliters of a horseradish peroxide conjugate of goat anti-mouse IgG (H + L), A, M (1:1500 dilution) was added to each well and incubated for 1 hr. at 20-22°C. After three washes with distilled water, the substrate OPD/hydrogen peroxidide was added and incubation continued for five to fifteen minutes. One hundred microliters of a stop solution (1 M hydrochloric acid) was then added and the absorbance at 490 nm read. Cultures which had an optical density reading greater than the control wells were removed to 2 cm² culture dishes, with the addition of normal mouse spleen cells in 1 x HT medium. After a further three days all of the 2 cm² cultures were rescreened for antibody and those testing positive again were cloned by limiting dilution. The cells in each 2 cm² culture were counted and cell concentration adjusted to 1 x 10⁵ cells per ml. The cells were diluted in complete medium and normal mouse spleen cells at concentrations of hybridoma cells of 5, 10 and 50 cells per ml added. The cells were plated into 96-well plates for each dilution. After 10 days the cloning plates were screened for growth. About 37% of all wells showed growth. The growth-positive wells were screened for antibody and those testing positive expanded to 2 cm² cultures and provided with normal mouse spleen cells. The cloning procedure was repeated 2 times until a stable antibody-producing hybridoma was obtained. At this point the cell culture was expanded from 2 to 9 to 75 to 150 cm² culture vessels, at which point ascite production could be commenced.

For ascites production, pristane primed IRCF1 female mice were used. 0.5 ml of pristane was injected intraperitoneally (IP) to each mouse, and the mouse allowed to rest fcr 10-60 days. At this time 4.5 x 10⁶ cells were injected IP into each mouse and ascites formed in 7-14 days. Ascites fluid was harvested with a pasteur pipette through a hole in the peritoneum.

### Reactivity of Monoclonal Antibodies

Monoclonals useful in practice in this invention include those which are specific for FIV and form a sufficiently strong interation with an FIV epitope, and an FIV antigen, to be useful in an immunoassay, for example, an ELISA to detect FIV antigen. In order to determine which of the above monoclonal antibodies are useful in this invention two main tests were used. The first was to determine whether the monoclonal antibody can bind FIV antigen and be detected with a conjugate of polycional antibody to FIV (an ELISA test, described in detail below). The second test was to form a conjugate of the monoclonal antibody with horseradish peroxidase and then determine whether the monoclonal antibody was able to compete with itself for FIV antigen, and whether it would compete with other monoclonal antibodies for that antigen. The latter test is useful to determine which monoclonals are useful in a mixture of monoclonal antibodies; generally antibodies with different reactivities will be combined together to allow detection of a larger range of FIV antigens in a sample. Such combination will greatly increase sensitivity.

Another test is to perform a Western blot to determine whether the monoclonal antibody has good reactivity with one or more FIV antigens. Generally, those monoclonals which show poor reactivity, that is, produce faint bands on the Western blot, are not suitable. Yet another test involves radioimmunoprecipitation assay (RIPA) where FIV virus labeled with ³⁵S-methionine is reacted with a monoclonal antibody to form within immunoprecipitate, and the immunoprecipitate run in a SDS-PAGE and autoradiographed to detect the labelled proteins. This analysis determines which of the monoclonal antibodies is able to detect precursor FIV proteins and not just mature proteins.

Examples of such tests are presented below, along with a description of the identification of various FIV antigens (or proteins) by which the monoclonal antibodies can be distinguished. These examples are not limiting to this invention.

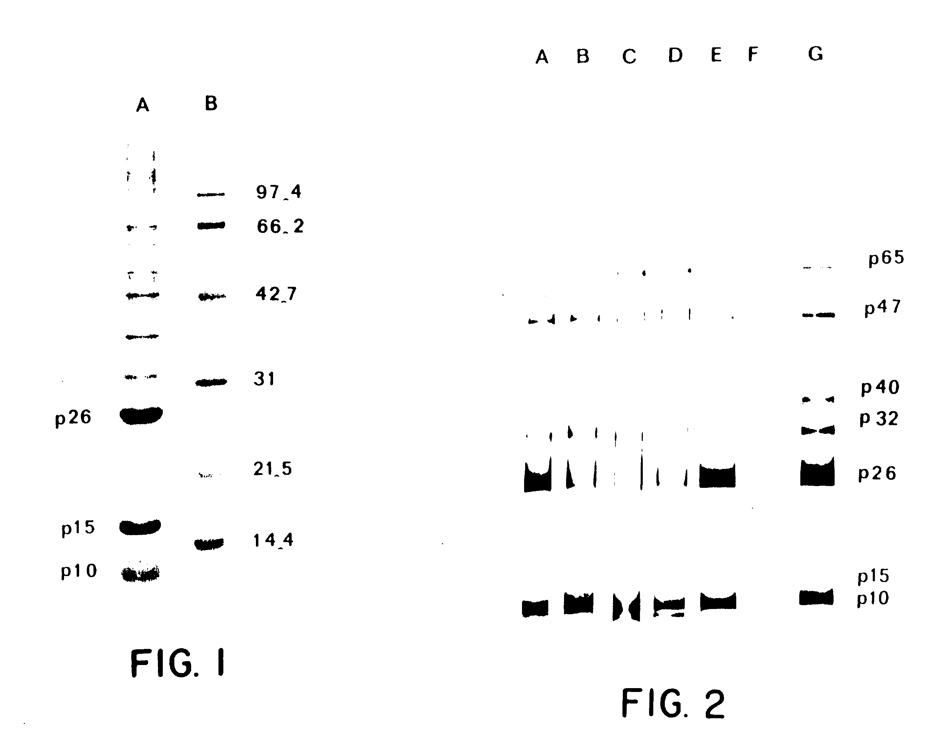

Referring to Fig. 1, proteins associated with purified FIV were analyzed by SDS/PAGE and compared with proteins isolated in an identical manner from the spent culture medium of uninfected cells. Analysis of the Commassie Blue stained gels revealed three major proteins with molecular weights of about 10, 15, and 26 kD, named p10, p15 and p26, respectively.

When an ELISA test was performed using disrupted FIV to identify cats possessing polyclonal antibody to FIV proteins, and Western blot analysis then performed on feline sera determined to be positive by ELISA, each of the cats had antibodies which reacted with one or more proteins or antigens of molecular weight p10, 15, 26, 40 and 65 kD under the conditions used.

Referring to Fig. 2, a standard Western immuno blot was performed as described by Towbin et al., 76 Proc. Natl. Acad. Sci USA 4350, 1979. Briefly, FIV was disrupted with SDS and proteins transferred to a sheet of nitrocellulose. The nitrocellulose sheet was blocked with 30% calf serum, 1% bovine serum albumin (BSA), and 0.05% Tween 20 in Dulbecco's phosphate buffer saline. The sheets were cut into 0.5 cm strips and incubated with a 1:100 dilution of serum sample in blocking buffer for 2 hrs. for 20-22°C. Strips were repeatedly washed with washing buffer (0.05% Tween 20 in Dulbecco's phosphate buffer saline) and then incubated with a second antibody (specific for feline heavy and light chain Ig) horseradish peroxidase conjugate (obtained from Kirkguard and Perry Laboratories Inc. Gaithersburg, MD). After 1 hr. incubation, the strips were repeatedly washed with washing buffer and incubated with the precipitating substrate 4-chloronaphthol for 10 min. The strips were partially dried and the results interpreted immediately. The serum in each of the lanes A-G was obtained from various cats infected with FIV. Predominant reactivity is detected with p26 and p15 and to a lesser extent with p10. Other proteins of 32, 40, 47 and 65 kD molecular weight are also detected.

Antibodies to glycoproteins can also be isolated. In particular, two glycoproteins of molecular weight 40 kD (gp40) and 130 kD (gp130) can be detected using PAGE and RIPA respectively. For example, an antibody (2F11) to envelope glycoproteins capable of recognizing antigenic sites possessed by the envelope precursor protein gp130 and the transmembrane protein gp40 was isolated as described below. Reactivity of this antibody with these proteins was determined by RIPA-PAGE analysis of ³⁵S-methionine/cysteine labeled FiV-infected cell lysates. The 130kD protein identified by the antibody was confirmed to be gp130 because it is sensitive to treatment with glycosidase H (which reduced the molecular weight of the glycoprotein to 75 kD). The 40kD protein detected was resistant to treatment by this enzyme.

2F11 antibody was isolated using mice-Balb/C (Amitek) and disrupted whole virus (Staph A buffer 1:1 for half an hour at 20°C-25°C). 300 µg of the antigen was mixed 1:1 with Freund's complete adjuvant and 300 µg boosts given at 2 week intervals with no adjuvant. Fusion of the cells was performed at 5 weeks, after initial immunization, with 2-300 µg boosts. Mice were rested for 3 days before the fusion. The fusion partner was FO ATCC CRL 1646 (J. Immunol. Methods 35:1-21, 1980). The resulting antibody was screened on fusion plates, rescreened, subcloned, and the subclones expanded for ascite production. The antibody is of subtype IgG₁ Kappa light chain.

Certain viral proteins, such as the gag (p26) antigens, are abundant in purified viral preparations, others such as the viral envelope proteins (gp130) tend to be lost during viral purification, and electrotransfer less efficiently for Western blot analysis than the gag antigens. Therefore, in order to more readily detect the viral envelope and the gag precurser proteins FIV cell extracts were labeled with ³⁵S-methionine and cysteine and examined by immunoprecipitation (RIPA). Confluent cultures of cells infected with FIV were incubated for 30 min. in methionine and cysteine-free Dulbecco's modified Eagle's medium. The cell cultures were then incubated for 4 hrs. in 8 ml of the same medium containing 100 microCuries per ml of ³⁵S-methionine and ³⁵S-cysteine (specific activity 1200 Curies per mM, New England Nuclear Corporation, Boston, MA). The radioactive tissue culture fluids were removed and the cells lysed with 5 ml of 10 mM sodium phosphate buffer pH 7.5 containing 100 mM NaCl, 1% Triton X 100, 0.5% sodium deoxycholate, 0.1% SDS, 0.1mM phenylmethylsulfonylfluoride, and 100 Kallikren inactivator units of aprotenin per ml. (Sigma Chemical Co., St. Louis, MO). Before use, the cell lysates were clarified by centrifugation 100,000 x g for 30 min. and the pellet discarded. Aliquots of the labelled cell lysates (0.1 ml) and 5 µl of serum being tested were mixed in a microcentrifuge tube and incubated for 1 hr. at 37°C and then overnight at 4°C. The next day, 0.2 ml of a 5% suspension of protein A Sepharose CL-4B beads (Pharmacia, Piscataway, NJ) in 10 mM of phosphate buffer, pH 7.5 containing 100 mM NaCl, 1% Triton X-100 and 0.1% SDS was added to each tube and mixed for 30 min. at 4°C. The antibody/antigen complexes bound to the protein A Sepharose beads were collected by centrifugation (2 min. at 20,000 x g) and washed 3 times in lysing buffer. The final pellet was resuspended in 25 µl SDS/PAGE loading buffer and heated and 100°C for 3 minutes. The Sepharose beads were removed by centrifugation and the supernatant applied to a PAGE. Gels were processed for fluorography using enlightening™ (New England Nuclear Corporation, Boston, MA) and exposed at -70° to Kodak XR-5 film, Sera from experimentally infected cats recognize proteins of 15, 22, 36, 40, 47, 110 and 130 kD. Although there were some quantitative and qualititative differences all cats appear to mount a response to p22, gp40, gp47 and gp130.

In order to determine which of the proteins identified by RIPA-PAGE analysis were related to the major internal structural protein, p26, RIPA-PAGE analysis was carried out using monoclonal antibodies which reacted with p26 as determined by Western blotting. This monoclonal immunoprecipited proteins p47, p36, p22 and p15. High molecular weight proteins (130 kD) of FIV which were not detected by the p26 specific monoclonal antibody. A protein of molecular weight 100 kD was also detectable utilizing serum antibodies obtained from some infected cats.

The monoclonals isolated as described above and tested by the above techniques were found to react specifically with p10, pl5, p26, p47 and to belong to the subtypes IgM, IgG1, IgG2, IgG3, IgG2A, and IgG23. Of these, some monoclonals reacted poorly with p26 and p47 and others reacted strongly or at an intermediate level. We found that the mcst significant test for usefulness of any specific antibody was the ELISA test. In a specific test, microtiter plates were coated with the anti-FIV monocional antibodies at 1 microgram per well in coating buffer (0.1 M NaHPO₄ pH 7.2). The monoclonal antibodies were incubated at 3°C overnight. The plates were then washed two times with phosphate buffered saline and 0.05% Tween-20 and the remaining liquid aspirated. The wells were then blocked with 1% bovine serum albumin in 0.1 M Tris pH 7 and this wash solution aspirated. FIV sample or a known FIV-antigen was then added by standard procedure. Horseradish peroxidase conjugate was prepared using either purified goat anti-FIV IgG or a second anti-FIV monoclonal antibody. Conjugate was then provided to the test wells. Following washing, a substrate-chromogen mixture (TMB/H₂O₂) was added to test wells and incubated for 15 minutes. Optical densities of wells at 650 nm was then determined. In this test a normal negative gave a result of approximately 0.06 O.D. units. A poor monoclonal antibody provided with 1 µg/ml of FIV antigen gave an O.D. reading of less than 0.5, cultures having a fairly good antibody yielded O.D. readings of 0.5 to 1.0. A good monoclonal antibody typically yielded an O.D. of greater than 0.2 when 1 ng per ml FIV was provided.

In a second experiment, each of the monoclonal antibodies was conjugated with horseradish peroxidase and analyzed in a competition assay for FIV antigen. In this way it was determined which monoclonal antibodies could be used in mixtures for standard immunoassays for FIV. Particularly good mixtures included the mixture of 2D4 or 3H8 (specific for p26), 4F2 (specific for pl5, p26 and p50) and 6E6 (specific for p26 and p50).

### Deposits

Cell lines producing monoclonal antibodies 3H8, 4F2 and 6E6 were deposited with ATCC on November 1, 1988 and given designation numbers HB9888, HB9889 and HB9890, respectively. The cell line producing monoclonal antibody 2F11 was deposited with the ATCC on November 17, 1989 and given designation number HB10295. The cell lines were deposited under the terms of the Budapest Treaty.

## Claims

1. A purified polypeptide comprising an epitope of a feline immunodeficiency virus (FIV) protein, said FIV protein being characterised in that it is:
a) an FIV envelope protein obtainable from FIV-infected cells;
b) approximately 130 kD in weight;
c) binds to monoclonal antibody produced by hybridoma cell line 2F11 (American Type Culture Collection (ATCC) Deposit Number HB 10295); and
d) binds to serum of FIV-infected cats.

2. A purified polypeptide according to Claim 1, in which said polypeptide has a weight of approximately 130 kD and binds to monoclonal antibody produced by hybridoma cell line 2F11 (American Type Culture Collection (ATCC) Deposit Number HB 10295).

3. A monoclonal antibody specific for an epitope of a polypeptide according to Claim 1 or Claim 2.

4. A monoclonal antibody according to Claim 3, wherein said monoclonal antibody is conjugated with a detectable label that does not significantly interfere with the specificity or affinity of said antibody for said epitope.

5. A monoclonal antibody according to Claim 4, wherein said detectable label is an enzyme.

6. A monoclonal antibody according to any of Claims 3 to 5, in which the monoclonal antibody cross-reacts with monoclonal antibody produced by hybridoma cell line 2F11 (ATCC Deposit Number HB 10295).

7. A monoclonal antibody according to Claim 6, wherein said enzyme is horseradish peroxidase.

8. A solution comprising a mixture of:
a) a monoclonal antibody according to any of Claims 2 to 7; and
b) at least one additional antibody which is selected from:
1) an antibody that recognises an FIV antigen of approximately 15 kD that binds to antibodies in serum of FIV-infected cats and is recognised by the monoclonal antibody produced by hybridoma cell line 4F2 (ATCC Deposit Number HB 9889);
2) an antibody that recognises an FIV antigen of approximately 26 kD that binds to antibodies in serum of FIV-infected cats and is recognised by monoclonal antibody produced by one or more of hybridoma cell lines 4F2 (ATCC Deposit Number HB 9889), 3H8 (ATCC Deposit Number HB 9888) and 6E6 (ATCC Deposit Number HB 9890) ;
3) an antibody that recognises an FIV antigen of approximately 47 kD that binds to antibodies in serum of FIV-infected cats and is recognised by monoclonal antibody produced by one or more of hybridoma cell lines 4F2 (ATCC Deposit Number HB 9889) and 6E6 (ATCC Deposit Number HB 9890); and
4) an antibody that recognises an FIV envelope protein of approximately 40kD that binds to antibodies in serum of FIV-infected cats and is recognised by monoclonal antibody produced by hybridoma cell line 2F11 (ATCC Deposit Number HB 10295).

9. A solution according to Claim 8, wherein the solution comprises the monoclonal antibody produced by hybridoma cell line 3H8 (ATCC Deposit Number HB 9888).

10. A solution according to Claim 8, wherein the solution comprises the monoclonal antibody produced by hybridoma cell line 4F2 (ATCC Deposit Number HB 9889).

11. A solution according to Claim 8, wherein the solution comprises the monoclonal antibody produced by hybridoma cell line 6E6 (ATCC Deposit Number HB 9890).

12. A solution according to Claim 8, wherein the solution comprises the monoclonal antibody produced by hybridoma cell line 2F11 (ATCC Deposit Number HB 10295).

13. A method for detecting an FIV envelope glycoprotein antigen in a sample, said method comprising the steps of:
a) providing an antibody that specifically recognises the polypeptide of Claim 1 or Claim 2;
b) contacting said antibody with said sample under conditions in which said antibody forms a complex with a sample antigen; and
c) detecting said complex, wherein the presence of said complex indicates the presence of said glycoprotein in said sample.

14. A method according to Claim 13, in which the FIV envelope glycoprotein antigen has a weight of about 130,00 kD and specifically binds to monoclonal antibody produced by hybridoma cell line 2F11 (ATCC Deposit Number HB 10295).

15. A method according to Claim 13 or Claim 14, wherein said antibody of step a) is a monoclonal antibody.

16. A method according to any of Claims 13 to 15, in which the antibody is part of a mixture as defined in any of Claims 8 to 12.

17. A method for detecting an antibody to FIV in a sample, said method comprising the steps of:
a) providing a purified polypeptide according to Claim 1 or Claim 2;
b) contacting said purified polypeptide with a sample comprising said antibody; and
c) detecting the presence of a complex formed between said polypeptide and said antibody, wherein the presence of said complex indicates the presence of said antibody in said sample.

## Patentansprüche

1. Gereinigtes Polypeptid umfassend ein Epitop eines Katzen-lmmunschwäche-Virus- bzw. FIV-Proteins, wobei das FIV-Protein dadurch gekennzeichnet ist, daß es:
a) ein FIV-Hüllprotein ist, das aus FIV-infizierten Zellen erhalten werden kann;
b) eine Masse von annähernd 130 kD besitzt;
c) sich mit einem monoklonalen Antikörper verbindet, der von einer Hybridomzellinie 2F11 (mit der Hinterlegungsnummer HB 10295 der American Type Culture Collection (ATCC)) produziert wird; und
d) sich mit dem Serum von FIV-infizierten Katzen verbindet.

2. Gereinigtes Polypeptid nach Anspruch 1, bei dem das Polypeptid eine Masse von annähernd 130 kD besitzt und sich mit einem monoklonalen Antikörper verbindet, der von der Hybridomzellinie 2F11 (mit der Hinterlegungsnummer HB 10295 der American Type Culture Collection (ATCC)) produziert wird.

3. Monoklonaler Antikörper, der für ein Epitop eines Polypeptids nach Anspruch 1 oder 2 spezifisch ist.

4. Monoklonaler Antikörper nach Anspruch 3, wobei der monoklonale Antikörper mit einem nachweisbaren Marker konjugiert ist, der die Spezifität oder Affinität des Antikörpers gegenüber dem Epitop nicht signifikant beeinträchtigt.

5. Monoklonaler Antikörper nach Anspruch 4, wobei der nachweisbare Marker ein Enzym ist.

6. Monoklonaler Antikörper nach einem der Ansprüche 3 bis 5, wobei der monoklonale Antikörper eine Kreuzreaktion mit einem monoklonalen Antikörper eingeht, der von der Hybridomzellinie 2F11 (ATCC-Hinterlegungsnummer HB 10295) produziert wird.

7. Monoklonaler Antikörper nach Anspruch 6, wobei das Enzym Meerrettich-Peroxidase ist.

8. Lösung, bestehend aus einer Mischung aus:
a) einem monoklonalen Antikörper nach einem der Ansprüche 2 bis 7; und
b) mindestens einem zusätzlichen Antikörper, ausgewählt unter:
1) einem Antikörper, der ein FIV-Antigen von annähernd 15 kD erkennt, das sich mit dem Antikörper im Serum von FIV-infizierten Katzen verbindet und von dem monoklonalen Antikörper erkannt wird, der von der Hybridomzellinie 4F2 (ATCC-Hinterlegungsnummer 9889) produziert wird;
2) einem Antikörper, der ein FIV-Antigen von annähernd 26 kD erkennt, das sich mit dem Antikörper im Serum von FIV-infizierten Katzen verbindet und von einem monoklonalen Antikörper erkannt wird, der von einer oder mehreren Hybridomzellinien 4F2 (ATCC-Hinterlegungsnummer 9889), 3H8 (ATCC-Hinterlegungsnummer HB 9888) und 6E6 (ATCC-Hinterlegungsnummer HB 9890) produziert wird;
3) einem Antikörper, der ein FIV-Antigen von annähernd 47 kD erkennt, das sich mit dem Antikörper im Serum von FIV-infizierten Katzen verbindet und von einem monoklonalen Antikörper erkannt wird, der von einer oder mehreren Hybridomzellinien 4F2 (ATCC-Hinterlegungsnummer 9889) und 6E6 (ATCC-Hinterlegungsnummer HB 9890) produziert wird; und
4) einem Antikörper, der ein FIV-Hüllprotein von annähernd 40 kD erkennt, das sich mit dem Antikörper im Serum von FIV-infizierten Katzen verbindet und von dem monoklonalen Antikörper erkannt wird, der von der Hybridomzellinie 2F11 (ATCC-Hinterlegungsnummer 10295) produziert wird.

9. Lösung nach Anspruch 8, wobei die Lösung den monoklonalen Antikörper enthält, der von der Hybridomzellinie 3H8 (ATCC-Hinterlegungsnummer 9888) produziert wird.

10. Lösung nach Anspruch 8, wobei die Lösung den monoklonalen Antikörper enthält, der von der Hybridomzellinie 4F2 (ATCC-Hinterlegungsnummer 9889) produziert wird.

11. Lösung nach Anspruch 8, wobei die Lösung den monoklonalen Antikörper enthält, der von der Hybridomzellinie 6E6 (ATCC-Hinterlegungsnummer 9890) produziert wird.

12. Lösung nach Anspruch 8, wobei die Lösung den monoklonalen Antikörper enthält, der von der Hybridomzellinie 2F11 (ATCC-Hinterlegungsnummer 10295) produziert wird.

13. Verfahren zum Nachweisen eines FlV-Hüllglykoprotein-Antigens, wobei das Verfahren folgende Schritten umfaßt:
a) Bereitstellen eines Antikörpers, der spezifisch das Polypeptid von Anspruch 1 oder Anspruch 2 erkennt;
b) Zusammenbringen des Antikörpers mit der Probe unter Bedingungen, bei denen dieser Antikörper einen Komplex mit einem Probenantigen bildet; und
c) Nachweisen dieses Komplexes, wobei das Vorhandensein dieses Komplexes auf das Vorhandensein des Glykoproteins in der Probe hindeutet.

14. Verfahren nach Anspruch 13, bei dem das FlV-Hüllglykoprotein-Antigen eine Masse von annähernd 130 000 kD besitzt und sich spezifisch mit dem monoklonalen Antikörper verbindet, der von der Hybridomzellinie 2F11 (ATCC-Hinterlegungsnummer 10295) produziert wird.

15. Verfahren nach Anspruch 13 oder Anspruch 14, wobei es sich bei dem Antikörper im Schritt (a) um einen monoklonalen Antikörper handelt.

16. Verfahren nach einem der Ansprüche 13 bis 15, bei dem der Antikörper Teil einer Mischung ist, wie sie in den Ansprüchen 8 bis 12 definiert ist.

17. Verfahren zum Nachweisen eines Antikörpers zu FIV in einer Probe, wobei das Verfahren folgende Schritten umfaßt:
a) Bereitstellen eines gereinigten Polypeptids nach Anspruch 1 oder Anspruch 2;
b) Zusammenbringen des gereinigten Polypeptids mit einer Probe, die den Antikörper enthält; und
c) Nachweisen des Vorhandenseins eines Komplexes, der zwischen dem Polypeptid und dem Antikörper entstanden ist, wobei das Vorhandensein des Komplexes auf das Vorhandensein des Antikörpers in der Probe hindeutet.

## Revendications

1. Polypeptide purifié comprenant un épitope d'une protéine du virus de l'immunodéficience féline (VIF), ladite protéine de VIF étant caractérisée en ce qu'il s'agit :
a) d'une protéine d'enveloppe de VIF pouvant être obtenue à partir de cellules infectées par VIF ;
b) ayant un poids d'environ 130 kD ;
c) se fixant à un anticorps monoclonal produit par la lignée cellulaire d'hybridome 2F11 (American Type Culture Collection (ATCC), numéro de dépôt HB 10295) ; et
d) se fixant au sérum de chats infectés par VIF.

2. Polypeptide purifié selon la revendication 1, dans lequel ledit polypeptide a un poids d'environ 130 kD et se fixe à l'anticorps monoclonal produit par la lignée cellulaire d'hybridome 2F11 (American Type Culture Collection (ATCC), numéro de dépôt HB 10295).

3. Anticorps monoclonal spécifique d'un épitope d'un polypeptide selon la revendication 1 ou 2.

4. Anticorps monoclonal selon la revendication 3, dans lequel ledit anticorps monoclonal est conjugué à un marqueur détectable qui n'interfère pas de façon significative avec la spécificité ou l'affinité dudit anticorps vis-à-vis dudit épitope.

5. Anticorps monoclonal selon la revendication 4, dans lequel ledit marqueur détectable est une enzyme.

6. Anticorps monoclonal selon l'une quelconque des revendications 3 à 5, dans lequel l'anticorps monoclonal réagit de façon croisée avec l'anticorps monoclonal produit par la lignée cellulaire d'hybridome 2F11 (numéro de dépôt ATCC HB 10295).

7. Anticorps monoclonal selon la revendication 6, dans lequel ladite enzyme est la peroxydase de raifort.

8. Solution comprenant un mélange de :
a) un anticorps monoclonal selon l'une quelconque des revendications 2 à 7 ; et
b) au moins un anticorps additionnel qui est choisi parmi :
1) un anticorps qui reconnaît un antigène de VIF d'environ 15 kD qui se fixe à des anticorps dans le sérum de chats infectés par VIF et est reconnu par l'anticorps monoclonal produit par la lignée cellulaire d'hybridome 4F2 (numéro de dépôt ATCC HB 9889) ;
2) un anticorps qui reconnaît un antigène de VIF d'environ 26 kD qui se fixe à des anticorps dans le sérum de chats infectés par VIF et est reconnu par l'anticorps monoclonal produit par une ou plusieurs des lignées cellulaires d'hybridome 4F2 (numéro de dépôt ATCC HB 9889), 3H8 (numéro de dépôt ATCC HB 9888) et 6E6 (numéro de dépôt ATCC HB 9890) ;
3) un anticorps qui reconnaît un antigène de VIF d'environ 47 kD qui se fixe à des anticorps dans le sérum de chats infectés par VIF et est reconnu par l'anticorps monoclonal produit par une ou plusieurs des lignées cellulaires d'hybridome 4F2 (numéro de dépôt ATCC HB 9889) et 6E6 (numéro de dépôt ATCC HB 9890) ; et
4) un anticorps qui reconnaît une protéine d'enveloppe de VIF d'environ 40 kD qui se fixe à des anticorps dans le sérum de chats infectés par VIF et est reconnu par l'anticorps monoclonal produit par la lignée cellulaire d'hybridome 2F11 (numéro de dépôt ATCC HB 10295).

9. Solution selon la revendication 8, dans laquelle la solution comprend l'anticorps monoclonal produit par la lignée cellulaire d'hybridome 3H8 (numéro de dépôt ATCC HB 9888).

10. Solution selon la revendication 8, dans laquelle la solution comprend l'anticorps monoclonal produit par la lignée cellulaire d'hybridome 4F2 (numéro de dépôt ATCC 9889).

11. Solution selon la revendication 8, dans laquelle la solution comprend l'anticorps monoclonal produit par la lignée cellulaire d'hybridome 6E6 (numéro de dépôt ATCC HB 9890).

12. Solution selon la revendication 8, dans laquelle la solution comprend l'anticorps monoclonal produit par la lignée cellulaire d'hybridome 2F11 (numéro de dépôt ATCC HB 10295).

13. Procédé pour détecter un antigène de glycoprotéine d'enveloppe de VIF dans un échantillon, le procédé comprenant les étapes consistant à :
a) dispositif d'un anticorps qui reconnaît spécifiquement la polypeptide de la revendication 1 ou 2 ;
b) mettre en contact ledit anticorps avec ledit échantillon dans des conditions dans lesquelles ledit anticorps forme un complexe avec un antigène d'échantillon ; et
c) détecter ledit complexe, dans lequel la présence dudit complexe indique la présence de ladite glycoprotéine dans ledit échantillon.

14. Procédé selon la revendication 13, dans lequel l'antigène de glycoprotéine d'enveloppe de VIF a un poids d'environ 130 000 D et se fixe spécifiquement à l'anticorps monoclonal produit par la lignée cellulaire d'hybridome 2F11 (numéro de depôt ATCC HB 10295).

15. Procédé selon la revendication 13 ou 14, dans lequel ledit anticorps de l'étape a) est un anticorps monoclonal.

16. Procédé selon l'une quelconque des revendications 13 à 15, dans lequel l'anticorps fait partie d'un mélange tel que défini dans l'une quelconque des revendications 8 à 12.

17. Procédé pour détecter un anticorps dirigé contre VIF dans un échantillon, ledit procédé comprenant les étapes consistant à :
a) disposer d'un polypeptide purifié selon la revendication 1 ou 2 ;
b) mettre en contact ledit polypeptide purifié avec un échantillon comprenant ledit anticorps ; et
c) détecter la présence d'un complexe formé entre ledit polypeptide et ledit anticorps, dans lequel la présence dudit complexe indique la présente dudit anticorps dans ledit échantillon.
